**Europäisches Patentamt**

**European Patent Office**

**Office européen des brevets**

(19)

(11) Publication number: **0 119 660**
**A1**

(12) # EUROPEAN PATENT APPLICATION

(21) Application number: **84200376.6**

(22) Date of filing: **15.03.84**

(51) Int. Cl.³: **A 61 B 5/10, A 61 B 6/00**

(30) Priority: **17.03.83 NL 8300965**

(43) Date of publication of application: **26.09.84**
**Bulletin 84/39**

(84) Designated Contracting States: **AT BE CH DE FR GB IT LI LU NL SE**

(71) Applicant: **Snijder, Nicolaas Roelof, Schooteindseweg 8A, NL-5756 BD Vlierden (NL)**

(72) Inventor: **Snijder, Nicolaas Roelof, Schooteindseweg 8A, NL-5756 BD Vlierden (NL)**
Inventor: **Snijder, Jan Gerardus Nicolaas, Dr., Schooteindseweg 8A, NL-5756 BD Vlierden (NL)**

(74) Representative: **Noz, Franciscus Xaverius, Ir. et al, Boschdijk 155 P.O. Box 645, NL-5600 AP Eindhoven (NL)**

(54) System of examining skeleton parts of a living body, more particularly the vertebral column of the human body.

(57) A system for examining skeleton parts of a living body comprising at least one primary imaging device for obtaining at a given instant a simultaneous image on a carrier or synchronous imaging signals related to a single image or imaging and position signals from the skeleton parts to be examined and at least one primary reference member related to a skeleton part to be examined, respectively, whereby the system furthermore comprises at least one secondary imaging device for obtaining during a given time images or imaging or position signals of the one primary reference member or primary reference members or of a secondary reference member related to the same skeleton part as the one primary reference member or of secondary reference members related to the same skeleton parts as the primary reference members, respectively.

ACTORUM AG

- 1 -

System of examining skeleton parts of a living body more particularly the vertebral column of the human body.

The invention relates to a system of examining skeleton parts of a living body comprising at least one primary imaging device for obtaining at a given instant a simultaneous image on a carrier or synchronous imaging signals, related to a single image, or respectively, imaging and position signals from skeleton parts to be examined and at least one primary reference member related to a skeleton part to be examined, respectively.

From Dutch Patent Application 7415910 there is known a device for making X-ray photographs of a patient in a standing position comprising a stand provided with a cassette container for an X-ray film cassette for examining the vertebral column of the human body or, in general, the body carriage.

This known device is provided with a foot plate on which the patient will stand, with at least three pressure sensors not being aligned supporting the foot plate, with an arithmetic unit connected with the pressure sensors for producing an output signal which is representative of the position of the vertical projection of the body's centre of gravity in the plane of the foot plate with respect to a reference point or the median and on the stand carrying the X-ray cassette with a mainly horizontally movable element and a vertical, wire-shaped member of X-ray absorbing material coupled with said element and located between the patient and the cassette container for occupying a position corresponding to the position of the median calculated by the arithmetic unit with respect to the sagital or frontal central plane corrected for the diverging beams of radiation.

The aforesaid known device is intended to simplify three-dimensional X-ray examination and has the advantage that the median is represented in the photographs so that judgment of the deviation of the vertebral column in the sagital and the orthogonal frontal plane is facilitated.

The known device thus permits of carrying out a three-dimensional examination of the vertebral column with only one X-ray tube and one cassette container, whilst in the same position of the patient X-ray photographs can be made in two orthogonal directions. Thereto the patient stands in order of succession in two positions differing by $90^{\circ}$ on the foot plate. Screening in the second position then waits until the median is again in the same position with respect to the sagital central plane as in the screening in the first position.

A disadvantage of the aforesaid known device is that only an instantaneous photograph of the condition of the vertebral column can be obtained under given conditions, whilst the number of successive instantaneous screenings has to be restricted due to adverse effect of a multiple exposure to X-rays required for making a suitable X-ray photograph. This disadvantage is more serious for young people in their period of growth.

The invention has for its object to obviate at least the above-mentioned disadvantage and to provide a system which enables of following the condition of the vertebral column and the positions of the vetebrae therein especially with respect to given reference points or lines and the like for a comparatively long time e.g. during physiotherapeutic treatment.

The invention provides a system of the kind set forth in the preamble which is characterized in that the system furthermore comprises at least one secondary imaging device for obtaining during a given time images or imaging or position signals of the one primary reference member or primary reference members or a secondary reference member related to the same skeleton part as the one primary reference member or of secondary reference members related to the same skeleton parts as the primary reference members, respectively.

The invention will be described more fully with reference to several embodiments with reference to the drawing in which:

Fig. 1 shows a system embodying the invention having a primary imaging device, particularly an X-ray photographing device and a secondary imaging device, particularly a video display device, as well as a measuring device for the weight distribution;

Fig. 2 is a detailed plan view of the supporting member of the weight distribution measuring device of Fig. 1;

Fig. 3 is a side elevation of the supporting member of Fig. 2 in which the stop members are indicated;

Fig. 4 is a side elevation of a part of a secondary imaging device and a tertiary reference member of a system in a further embodiment of the invention.

Fig. 5 is a plan view of the apparatus shown in Fig. 4;

Fig. 6 shows a device for localising the seventh cervical vertebra and the fifth lumbar vertebra by means of mechanical sensors;

Fig. 7 is a plan view of the device of Fig. 6;

Fig. 8 shows a device for localising the seventh cervical vertebra and the fifth lumbar vertebra by means of acoustical or light waves;

Fig. 9 is a plan view of the device of Fig. 8;

Fig. 10 shows a blockdiagram of a further embodiment of the system according to the invention; and

Fig. 11 shows a stabiloscope.

In the first place the field of application of the present invention will be dealt with in general terms before going over to the details of this invention.

The phenomenon with the growing youth to correct any signalled difference in lengths of legs by means of higher heels, is a phenomenon almost even more disquieting than the tendency to prescribe an arch support for everyone having back complaints and for every foot having a less smooth arch. The same applies to the prescription of physiotherapy in the event of painful disturbances inside the muscular system. Nevertheless the choice of a shoe, an arch support and the physiotherapy may be particularly important to obviate existing complaints, to stem or correct statical deviations and/or deformations. The question is indeed what objective parameters and what objective checking systems are available to fix the indication and the final effect and to evaluate the next objective norm. After the device known from Dutch Application 7415910 was taken into use, hundreds of records on X-ray photographs of the position of the vertebral column and the pelvis with respect to the median and the centre of the X-ray cassette corresponding to the centre of the foot plate, particularly for young people having a different lengths of legs were obtained. After these primary examinations control-examinations were made for comparison with a correction of the leg shortening and hence the horizontal position of the pelvis. In various cases direct improvement of the static occurred, that is to say the position of the vertebral column with respect to the median, but in almost as may cases improvement was not obtained and even a further group showed a deterioration of the static. It has taken much time and research work before it became obvious which conditions had to be fulfilled to get at a normally predictable reaction of the shape and position of the vertebral column in correcting length differences of legs. In the course of years it became obvious that in correcting differences in leg lengths only very large differences with respect to a starting position could be judged quantitatively by visual observation alone. An explanation of these unpredictable reactions of the body was finally found in such concepts as tolerancies of muscles, ligaments, sinews and joint-ligaments. Apart from these factors which directly influence the static, the central nervous system plays an important part via afferent pulses from the periphery or just the absence thereof. Disturbances in the tolerancies of the abovementioned anatomic structures may be permanent or correctable after a shorter or longer time.

As stated above the practical use of the device known from Dutch Patent Application 7415910 is seriously inhibited by the fairly high dose of X-rays required for a photograph of 90 x 30 cms at a distance of 3 ms. The average voltage is 90 kV at an exposure time of 0.5 to 1 sec. In organizing a training programme or a scheme only one and at the most, three X-ray information sources are available. Intermediate controls cannot be accounted for within short period of time, the more so since young children in full growth are concerned here, where the effect of X-rays may be more damaging than in adults.

It should be noted that the median shown divides the body only seldom into two equal parts owing to the displacement of the body to the left or to the right and to unequal load of the foot so that the resultants of forces elft and right to the centre (diagonal line) of the floor plate are unequal (see Fig. 11).

The invention now relates to a system of examining skeleton parts of a living body, in particular the vertebral column of the human body which comprises at least one primary imaging device for obtaining at a given instant a simultaneous image on a carrier or synchronous imaging signals related to a single image or imaging and position signals of the vertebral column and at least one primary reference member related to a given vertebra. This primary imaging device may be an X-ray circuit including an X-ray source and an X-ray cassette. The carrier, in this case the X-ray photograph will give an image of the separate vertebrae of the vertebral column and at least one mark on one of the vertebrae. It is also possible to use a different primary imaging device, for example one using another type of penetrating radiation e.g. gamma rays or accoustic energy e.g. ultrasonic energy. By a receiver of the radiation or the accoustic energy an image of the vertebral column is obtained on a monitor. The electronic circuit should have a memory since the photograph is made once and within a very short time because of the noxious radiaton. This does not apply to the use of ultrasonic energy. However with a view to the field of application of the invention the use of only primary ultrasonic imaging devices would be too expensive and in the present state of the art it may be doubted whether as good an image can be obtained as with the use of noxious penetrating radiation.

The primary imaging device makes an image not only of the vertebral column; at given places of the skin related to given vertebral bodies primary reference members are applied which can be imaged by the radiation or energy used by the primary imaging device. In the case of an X-ray device the X-ray photograph shows at least the vertebral column and primary reference members related to given vertebrae. In the case of other primary imaging devices the imaging signals are representative of the vertebral column and the associated primary reference members. In the latter case it is not necessary for the primary reference members to provide imaging signals for example when using ultrasonic energy it will be ultrasonic energy signals. But is may also be position signals emanating from position sensors coupled with the primary reference members giving off position signals which are representative of the position of a primary reference member. By means of electronics the imaging signals of the vertebral column are combined with the position signals of respective vertebral bodies so that a combined image is displayed on the monitor.

A further characteristic of the invention is that the device comprises at least one second imaging device for obtaining images for a given period of time or imaging or position signals of one or more primary reference member(s) or one or more secondary reference member(s) related to the same vertebra(e) as the primary reference member(s), respeciveli

It is important to note that in the secondary imaging device no X-rays or other penetrating radiation is used. In the secondary imaging device accoustic energy is used inter alia ultrasonic signals, light signals e.g. laser rays or other electromagnetic radiation. A further possibility resides in that the secondary imaging device uses at least partly position sensors producing position signals.

The secondary imaging device may for example comprise a video camera which provides a position signal of the primary reference members and/or secondary reference members associated with respective skeleton parts, particularly vertebral bodies. Then moving images of these reference members are obtained.

Like in the device known from Dutch Application 7415910 the system according to the present invention is provided with in this case at least one weight distribution measuring device having a primary and/or secondary imaging device associated with it. The weight distribution measuring device comprises a supporting member for supporting foot members on which is bearing the human body whose vertebral column has to be examined. The foot members may be the feet of the human body but also the legs of a chair on which the person to be examined is sitting or the foot members of a saddle or the like. With the supporting member is mechanically connected a sensor member for producing at least one control signal which is representative of the weight distribution of the body among the foot members for example the feet of combinations thereof for example every two legs of a chair and a medain indicating member for producing position signals representative of the median i.e. the perpendicular line from the centre of gravity to the supporting member or for driving a movable tertiary reference member in response to the control signal when used in an X-ray circuit, for example a vertical wire-shaped member of a material absorbing X-rays. At any rate in the X-ray photograph a line occurs and in a monitor image of the first imaging device there is a line which is stationary which is representative of the position of a vertebral body, the marking of which is also stationary. However, in the image of the second display device on the monitor the line representative of the centre of gravity will be movable as well as the position marking of respective vertebral bodies.

For calibrating the primary and secondary imaging devices a further line can be displayed in the monitor image or be generated therein.

As in the device described in Dutch Patent Application 7415910 the X-ray cassette may be disposed so that its centre corresponds with the central line of a foot plate of the supporting member so that the indication of the centre on the X-ray photograph is related to the central line of the foot plate. When a different imaging technique is used for the primary imaging device, there may be provided a further reference member which can be reproduced or the image of which can be generated on the monitor from position data. This also applies to the secondary imaging device.

Hereinafter the system according to the invention will be further explained with reference to practical embodiments shown in the drawings.

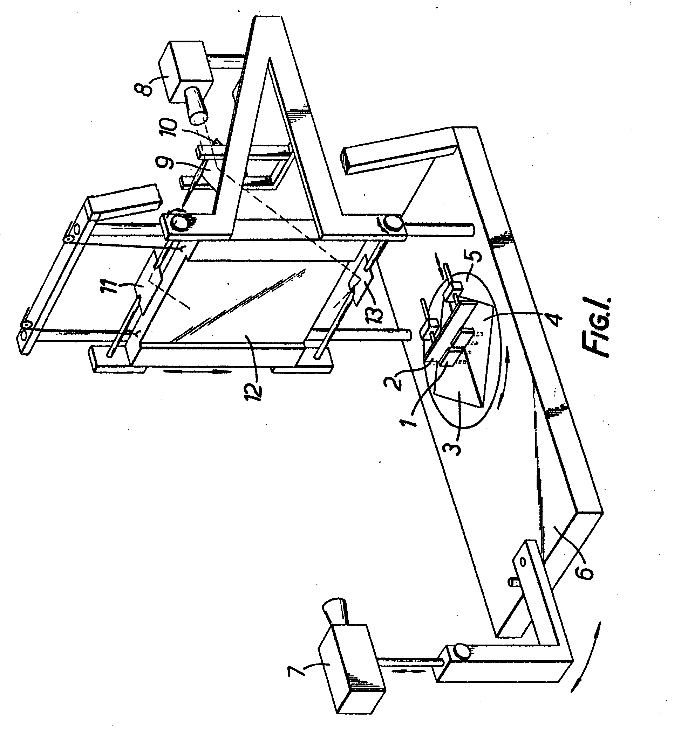

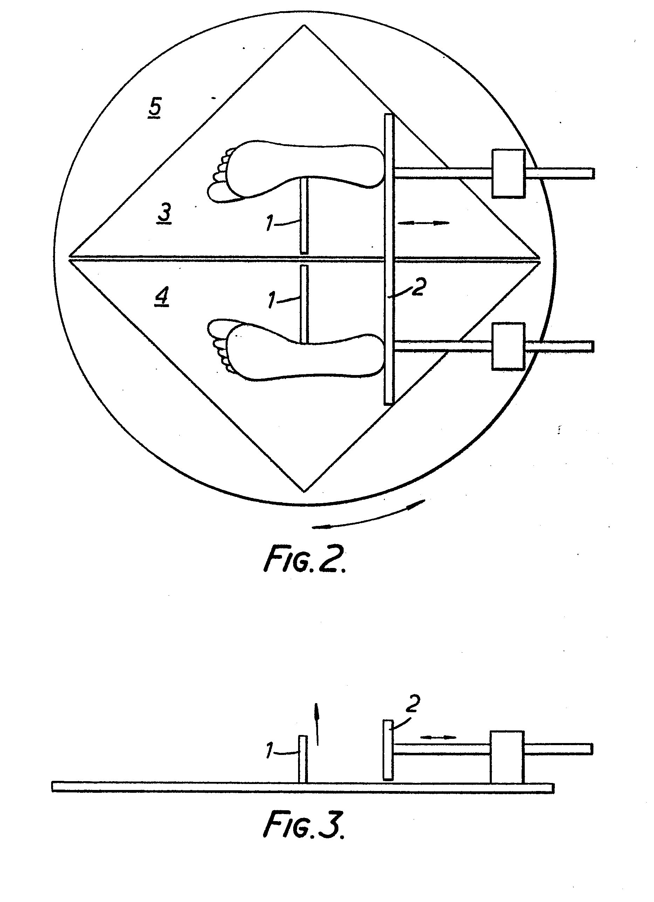

Fig. 1 of the drawings shows a first embodiment of the system in accordance with the invention. This system comprises a single primary imaging device and a single secondary imaging device which are in fact partly combined and weight distribution measuring device. Only the cassette container 12 of the X-ray circuit of the primary display device is shown. It is schematically indicated that the cassette container is movable in a vertical direction. The weight distribution measuring device comprises a round foot plate 5 in which the recording system is arranged, the plate 5 forming part of a much larger platform 6 to give sufficient safety to the person in his upright position. From this constant starting position the circular platform can turn through an angle of $90^{o}$ to the left and to the right whilst at the two extreme positions a fixation system again ensures a reproducible position of the person standing on it. The circular platform 5 has a rhomb divided into two isosceles triangles 3, 4, each of which bears independently of the other on three pressure sensors arranged at the corners. Recording of the pressure may in principle be performed by piezo-electrically    (quartz), but preferably by means of strain gauge bridges. In contrast to the weight distribution measuring device in the Dutch Patent Application 7415910, wherein recording is performed by means of three pressure sensors disposed in a single triangle the device embodying the invention uses two independently operating triangles having each three sensors i.e. in total six sensors. Moreover the disposition of the patient on the weight distribution measuring device is quite different from that of the known device. In the known device the feet are standing up against a triangular stop to the front and the rear side has a fixed stop at the heel. Due to this heel stop, in case of an X-ray photograph in lateral direction there will be a great difference between the recorded place of the centre of gravity of a large man's foot and that of a small child's foot. This situation on the weight distribution measuring device will be further discussed with reference to the Figs. 2 and 3.

The secondary imaging device is a video display device.

Referring to Figs. 1, 4 and 5 the secondary imaging device will be further explained. The secondary imaging device comprises a displaceable mirror 11 directed to the seventh cervical vertebra C7 of the person 20 to be examined and a second adjustable mirror 13 is directed to the fifth lumbar vertebra L5 of the person to be examined. There is furthermore a fixed mirror 9 directed towards the adjustable mirror 11, whilst a fixed mirror 10 is directed to the adjustable mirror 13. By means of the optical parts 9, 10, 11 and 13 and a video camera 8 display signals are obtained from primary or secondary reference members (not shown) fastened to the skin for example at the seventh servical vertebra and the fifth lumbar vertebra which members may also be formed by marks made by ink. Marks made on the seventh cervical vertebra and the fifth lumbar vertebra serve to record the equilibrium in the front-to-rear direction. Viewed from aside other reference points have to be used such as for example the auditory canal and the ear-lobe for localising the neck, the shoulder and the hip.

The tertiary reference member comprises a wire of lead 14. The lead wire 14 is represented by the primary X-ray display device on a sensitive plate.

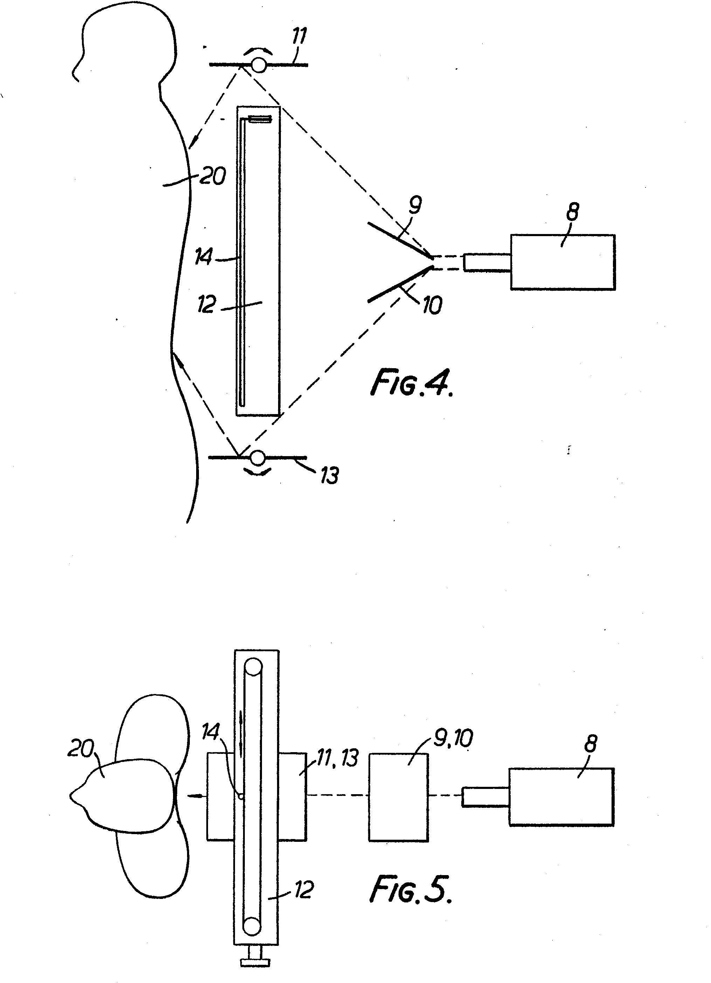

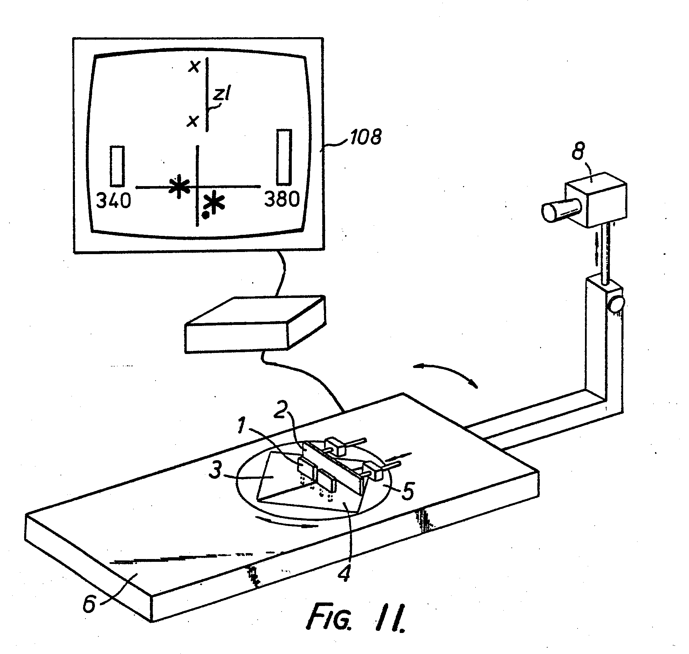

Fig. 5 clearly shows that the lead wire is driven by a rope passing around pulleys. The rope drive (not shown in detail) will be described more fully with reference to Fig. 10.

From the position signals of the reference members an image is generated. The line is generated from position signals emanating from the sensors via the computer.

Referring now to Figs. 2 and 3 as an anatomic point of orientation applies the prominent part of the navicular to the foot providing a division of the foot of 1 : 2, respectively the sides of teh heel and the forefoot. Thus both in front-to-rear direction and in lateral direction the condition of an identical load is fulfilled independently of the dimensions of the foot of a person. As a second important difference it applies that spontaneous disposition of the feet is possible and, moreover, any other position as desired by the examiner. It is apparent that the influence of the shape and the position of the foot is important with respect to human static. For the stop of the navicular two aligned vertical plates 1 are used each on one of the triangles at right angles to the line of separation between the triangles. Dependent on the lenght of the person examined and the desired higher stability these plates may have other lengths and places. In this manner the distance between the two bone extensions may vary between 5 and 20 cms. If once the position is occupied a displaceable heel stop 2 is moved along its guides and fixed in place. In this way identical load is ensured after the platform has been turned through an angle of $90^\circ$.

From the inoperative part of the platform is built up the vertical stand comprising inter alia the X-ray cassette, the lead wire and the motors required for displacing the same. To this stand are furthermore fastened the mirrors 9, 10, 11 and 13. The primary and/or secondary reference members may be formed by a push-button joint. These members need not be metal knobs, they may have the form of a short line.

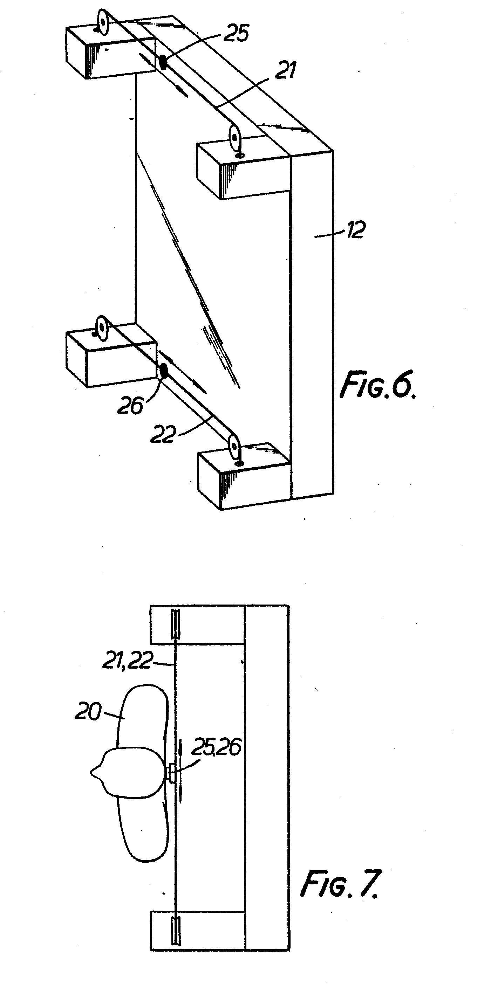

Figs. 6 and 7 show part of a secondary display device provided with means for producing position signals relating to the primary and/or secondary reference members 25 and 26 respectively associated with the places of the seventh cervical vertebra and the fifth lumbar vertebra respectively, connected by a wire movable in three directions with two potentiometers connected to a differential circuit. Reference numeral 12 designates the cassette container and 20 the person to be examined. Reference members 25 and 26 may be stuck to the skin by means of an adhesive.

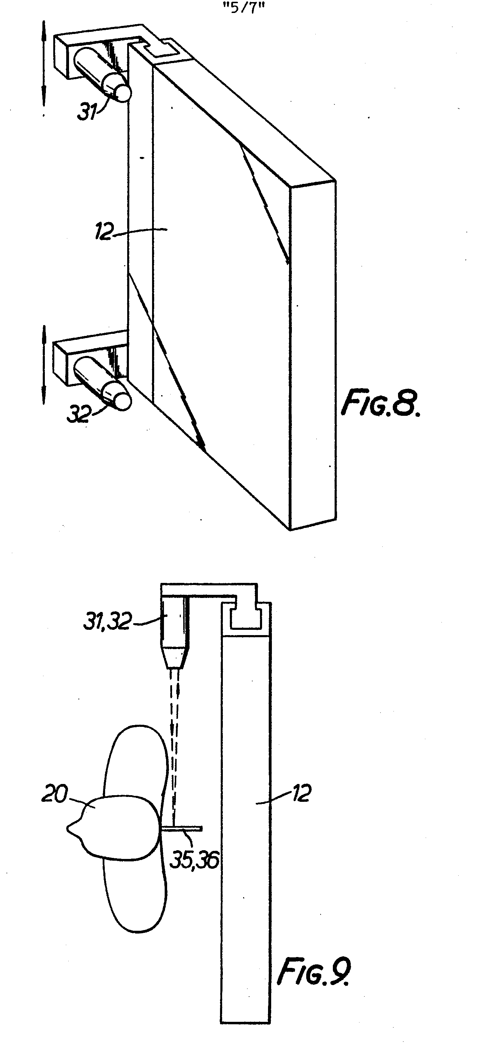

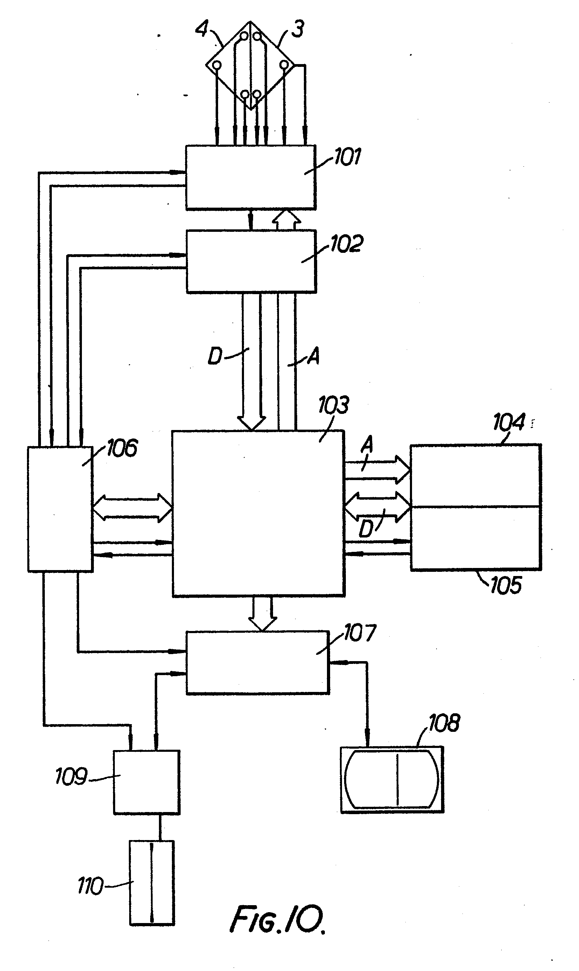

Figs. 8 and 9 show part of a secondary imaging device for producing imaging signals with respect to primary or secondary reference members associated with the seventh certical vertebra and the fifth lumbar vertebra. Reference numerals 31 and 32 designate transceivers for sound, light or electromagnetic waves. The primary or secondary reference members for indicating the place of the seventh cervical vertebra and the fifth lumbar vertebra have the shape of a reflection plate 35, 36.

Fig. 10 shows in a blockdiagram the simplest form of a system according to the invention. Reference numerals 3 and 4 again designate the right-hand and left-hand pressure plates each having three pressure sensors disposed at the corners of the triangles.The sensor member including the six pressure sensors is connected with programmable switches 101 connected by means of an analogue/digital converter 102 to arithmetic unit 103. The arithmetic unit 103 is connected to a memory 104 and a programme memory 105 as well as to a control device 106. The control unit 106 is furthermore connected to the programmable switches and the analogue/digital converter. The arithmetic unit and the control unit are furthermore connected with an interface 107 which in turn, is connected to a monitor 108 and on the other hand to a control unit for the lead line 109. Reference numeral 110 designates the photosensitive plate with a reproduction of the lead line which is displaced with respect to the central line, whilst the monitor shows the median by means of position signals which is also shifted with respect to the central line of the image corresponding with the central line of the photosensitive plate. The screen may display the weight distribution on the Left and on the Right in columns and numbers (centre of gravity left; centre of gravity right (*,*) Fig. 11).

In the above described embodiments, which constitute an implementation of the simplest form of the system according to the invention there is always referred to a single primary imaging device, a single secondary imaging device, a weight distribution measuring device and a tertiary reference member associated with the latter. In practice a hospital will have a primary imaging device for example an X-ray circuit or a sonograph operating on penetrating radiation for obtaining a reproduction of the vertebral column of the person to be examined. With various physiotherapeuts or manual therapeuts several separate secondary imaging devices may be used with a weight distribution measuring device, a tertiary reference member and a calibration member for indicating, for example, the centre of the X-ray photograph and the centre of the image on the monitor. These secondary imaging devices may be calibrated to the single primary imaging device of the hospital. The hospital can now provide an X-ray photograph or through a memory store the possibility of displaying on the monitors of the various secondary imaging devices of the instananeous picture taken by the primary imaging device of the hospital of the condition of the vertebral column of the patient with reference marks. This information can be stationarily displayed on the monitor. During a physiotherapeutic treatment the patient can stand on the weight distribution measuring device of the respective secondary display device and as the case may be at the same time with the stationary picture the monitor can show during the period of treatment a moving picture of the representative of the approximation of the median and the position marking of the seventh cervical vertebra and the fifth lumbar vertebra, for example the weight distribution and the centre of gravity to the left or to the right respectively so that the physiotherapeut can observe the effect of his treatment during his work and after various treatments.

In the display of the vertebral column it is preferred to display also the pelvis and part of the upper legs. X-ray films of 90 x 30 cms do permit this without any difficulty.

Apart therefrom it is possible with the aid of a second video camera (Fig. 1) or another detection system for example a laser system to display contours of the patient on the monitor at the side of the above-described images, as the case may be two elevational views. The secondary imaging devices positioned near the physiotherapeut together with the tertiary reference member, if any, the weight distribution measuring member and a display device or monitor may together be termed stabiloscope. Fig. 11 shows a stabiloscope with a display device 108, the same reference numerals being used as in Fig. 1. The difference between the disposition of Fig. 1 and that of Fig. 11 is that in Fig. 11 no X-ray circuit is available so that the associated means 9 to 13 are dispensed with. The video camera 8 can be arranged in the same manner as the video camera 7 in Fig. 1. By means of the weight distribution measuring device comprising the parts 1 to 5 suitable electronic circuits produce a reference line, the above-mentioned median on the screen of the display device as well as a line, the above described central line indicating the centre of the foot plate 5 on the platform 6. The shape of the contact surface between the parts 3 and 4 of the foot plate and the respective foot are also determining the weight distribution across the foot plates and hence the position of the generated median. As stated above this weight distribution may be represented in the form of columns of proportional magnitudes and/or numerical values at a suitable place, for example symmetrically on both sides of the median generated on the screen. In particular the secondary reference members may be of the kind such that with the aid of the video camera 8 only position signals are produced, for example, reference member in the form of a dash code and the position signal in the form of a pulse sequence. The image to appear on the display device is then completely generated by the computer. On the screen of the display device 108 in Fig. 11 are indicated: median zl, mass centre of gravity ●, detected vertebrae x, weight distribution i.e. right-hand and left-hand point of engagement* and weight columns ▯. For that matter the invention is not restricted to the above described mode of producing a picture.

The stabiloscope thus permits of simultaneously detecting a plurality of vertebrae in the front-to-rear direction. In a lateral direction the reference points to be detected are: the auditory canal, approximately corresponding with the place of the dens or the ear lobe which has the advantage that the reference member can be readily attached thereto, given parts of the shoulder, the pelvis, the knee, for example, the joint gap thereof. The reference members to be detected are such that recognition by the video camera 8 i.e. recognition of picture and pattern or position determination are possible, for example, by different dash codes, after which via the computer a predetermined sign, for example, an X or an O or a piece of line can be generated on the monitor. Apart from the references discussed above the monitor may show a cross of two orthogonal, intersecting lines corresponding to the diagonals of the rhombic foot plate with respect to which a movement of the generated median is generated in the form of a moving sign as well as the right-hand and left-hand point of engagement.

Preferably the video camera 8 is vertically adjustable which has the advantage that the video camera 8 can be placed nearer the platform 6 so that with a view to different lenghts of test persons it is not necessary to scan the whole range at one time. As stated above the foot plate may be rotatably arranged to carry out two orthogonal observations with the secondary imaging devices. As an alternative the foot plate may be stationary and the video camera 8 may be supported so that it can turn through $90^{\circ}$ or precautions may be made to fix the video camera onto different supports which provide positions of the video camera at an angle of $90^{\circ}$ to one another. Instead of using a vertical adjustability of the video camera 8 two vertically spaced apart video cameras may be used which together cover the whole lenght of any test person. In general this all not only applies to the stabiloschope but to all sytems described above embodying the invention.

Finally the distance between the video camera and the test person may be adjustable, whilst the video camera could be turned, if desired, through $180^{\circ}$.

Also because of the noxious X-rays in multiple tests many results of orthopaedic treatments have not been processed statisically, for example, the position of the foot and the influence thereof on the human equilibrium and the influence of concervative or operative treatments on the human equilibrium. In this respect it is noted that the primary imaging device may remain important in connection with the recorded or stored position on the basis whereof certain measures could or should be taken. According to the invention it will be possible after examination and recording of a great plurality of patients to programme the computer so that primarily the cause of the disturbed balance is calculated and demonstrated by examination on stabiloscope. This particularly applies to the examination of disturbances in the stereotypes, a term meaning the physiological pattern of consecutive muscular actions as a result of movement in particular in the shoulder joints and the pelvis joints. It is thus essential to obtain information with respect to static and dynamic. Apart from the possibility of storing data in the memory of the computer it may be effective to use a printing device to record an instantaneous picture from the monitor screen on paper whilst in addition storage in the computer memory or on a different record carrier, for example, magnetic disc can be maintained. Including a stabiloscope in an X-ray circuit permits stabilo-radiology.

The present invention thus relates to recording deviations from the human equilibrium in a standing position. Apart from the seventh certical vertebra and the fifth lumbar vertebra also the axis, the twelfth thorax vertebra, the first lumbar vertebra, the pelvis comb hight, the rotation of the pelvis and the symphysis may be observed by adding thereto primary and/or secondary reference members or marks.

The figures used in the claims are only meant to explain more clearly the intention of the invention and are not supposed to be any restriction concerning the interpretation of the invention.

- 1 -

CLAIMS

1.    A system for examing skeleton parts of a living body comprising at least one primary imaging device for obtaining at a given instant a simultaneous image on a carrier or synchronous imaging signals related to a single image or imaging and position signals from the skeleton parts to be examined and at least one primary reference member related to a skeleton part to be examined, respectively, characterized in that the system furthermore comprises at least one secondary imaging device for obtaining during a given time images or imaging or position signals of the one primary reference member or primary reference members or of a secondary reference member related to the same skeleton part as the one primary reference member or of secondary reference members related to the same skeleton parts as the primary reference members, respectively.

2.    A system as claimed in Claim 1, characterized in that it furthermore comprises at least one weight distribution measuring device comprising a supporting member for supporting foot members supporting the body whose skeleton parts have to be examined, a sensor member mechanically connected with the supporting member for producing at least one control signal which is representative of the weight distribution of the body among the foot members or combinations thereof and a median indicating member for producing in response to the control signal or control signals position signals representative of the median.

3.    A system as claimed in Claim 1 or 2, characterized in that it furthermore comprises a calibrating member related to a central line of an image carrier to be used in a primary imaging device and/or of an image to be formed by a primary and/or secondary imaging device, and providing respective position signals.

4. A system as claimed in Claims 2 and 3, characterized in that a supporting member is provided with a foot plate and in that the calibrating member is related to the central line of the foot plate.

5. A system as claimed in Claim 2 or 4, characterized in that the foot plate consists of two parts and the sensor member comprises an even number of at least six pressure sensors, half of them co-operating with the respective part of the foot plate and not being in line.

6. A system as claimed in Claim 5, characterized in that the foot plate comprises two isosceles triangles, the bases of which are located near one another and the six pressure sensors are arranged at the corners of the respective triangles, the foot members being the feet of the body to be examined the foot plate being furthermore provided with a single heel stop member and two further stop members for the navicular of the respective foot whereby the feet have to be placed one on each side of the two neighbouring bases of the triangles.

7. A system as claimed in anyone of the preceding Claims, characterized in that the skeleton parts to be examined comprise the vertebral column of in particular the human body and in that at least one of the primary and/or secondary reference members is applied to that part of the skin which is associated with a respective vertebra of the column to be examined.

8. A system as claimed in Claim 7, characterized in that a first primary and/or secondary reference member is associated with the seventh cervical certebra and a second primary and/or secondary reference member is associated with the fifth lumbar vertebra.

9. A system as claimed in anyone of the preceding Claims, characterized in that a primary imaging device comprises a source of penetrating radiation or a source emitting acoustic energy.

10. A system as claimed in anyone of the Claims 1 to 8 and 9, characterized in that a primary imaging device is provided with an X-ray source and an X-ray cassette, the body to be examined being disposed between the source and the cassette.

11. A system as claimed in anyone of the Claims 2 to 10, characterized in that a tertiary reference member is a lead line suspended vertically and being horizontally movable.

12. A system as claimed in Claim 11, characterized in that the lead line is suspended between the body to be examined and the X-ray cassette.

13. A system as claimed in anyone of the preceding Claims, characterized in that a secondary imaging device is provided with a source of acoustic, visible or non-visible light or electromagnetic energy, the position of the primary and/or secondary reference members being determinable with the aid of at least one of these energies.

14. A system as claimed in Claim 13, characterized in that a secondary imaging device is provided with at least one video camera and, if necessary optical members co-operating therewith or at least one ultrasonic transceiver.

15. A system as claimed in Claim 14, characterized in that the relative positions of the camera(s) and the foot plate of the weight distribution measuring device are variable.

16. A system as claimed in anyone of the preceding Claims, characterized in that it comprises at least one display device or monitor for displaying or generating an image or images of at least one of the primary or secondary imaging devices.

17. A system as claimed in anyone of the preceding Claims, characterized in that at least a primary and/or secondary imaging device is provided with a memory for a single image or signals relating thereto.

18. A stabiloscope, characterized in that it comprises at least one secondary imaging device and a weight distribution measuring device as claimed in any respective one of the preceding Claims, and a display device or monitor.

19. A stabiliscope as claimed in Claim 18, characterized in that the display device provides continuous information with respect to the static of a person by alpha-numeric and/or graphic display of at least one of the following items:

right-hand, left-hand and resultant centre of gravity, median, reference members and weight distribution (Fig. 11).

Fig.I.

0119660

FIG.2.

FIG.3.

0119660

FIG.4.

FIG.5.

FIG.6.

FIG.7.

0119660

*Fig.8.*

*Fig.9.*

Fig.10.

FIG. 11.

0119660

European Patent
Office

**EUROPEAN SEARCH REPORT**

Application number

EP 84 20 0376

## DOCUMENTS CONSIDERED TO BE RELEVANT

| Category | Citation of document with indication, where appropriate, of relevant passages | Relevant to claim | CLASSIFICATION OF THE APPLICATION (Int. Cl. ³) |
|---|---|---|---|
| A,D | NL-A-7 415 910 (J.G.N. SNIJDER)<br><br>* Page 3, line 2 - page 4, line 9; page 5, line 13 - page 6, line 4; page 7, lines 4-17; page 8, lines 9-16; page 9, lines 1-12; page 11, lines 1-19; figures 1-5 * | 1-4,9-13 | A 61 B 5/10<br>A 61 B 6/00 |
| A | DE-A-2 158 457 (A.G. BROWN, BOVERI & CIE.)<br>* Page 3, lines 11-18; page 7, line 21 - page 8, line 16; page 9, lines 5-18; page 12, lines 5-23; figures 1-4 * | 1,2,5 | |
| A | DE-A-3 022 994 (K. SCHMIEDECKER)<br><br>* Page 1, claims 1-3; page 7, line 13 - page 9, line 7; figure 1 * | 1,3,7,8,13,17 | **TECHNICAL FIELDS SEARCHED (Int. Cl. ³)**<br><br>A 61 B |
| A | DE-A-2 833 621 (G.B. GUTMANN)<br><br>* Pages 1,2; claims 1-4; page 14, line 15 - page 17, line 22; page 19, lines 12-20; page 22, line 18 - page 23, line 15; figures 1,4-9 * | 1,3,7,9-13 | |

-/-

The present search report has been drawn up for all claims

| Place of search<br>THE HAGUE | Date of completion of the search<br>25-06-1984 | Examiner<br>RIEB K.D. |
|---|---|---|

# EUROPEAN SEARCH REPORT

European Patent Office

Application number

EP 84 20 0376

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl. ³) |
|---|---|---|---|
| A | DE-A-2 235 434 (PREMED GmbH) <br> * Page 2, line 5 - page 3, line 5; figure 1 * | 2,5 | |
| A | US-A-3 256 611 (R.L. DEMING) <br><br> * Column 1, lines 28-39; column 3, line 50 - column 4, line 5; column 6, lines 1-28; figures 1-10 * | 1,3,4, 9-11, 17 | |
| A | US-A-3 514 606 (G.P. RABEY) <br><br> * Column 1, lines 56-63; column 2, lines 16-22; column 3, lines 6-37; column 6, lines 39-45; column 11, lines 2-20; figures 1,17-30 * | 1,3,4, 9-12, 17 | |
| A | FR-A-1 564 781 (COMP. GEN. D'ELECTRONIQUE INDUSTRIELLE LEPAUTE) <br> * Page 2, column 1, lines 22-55; figure 1 * | 1,3,4, 13,14, 16,17 | |

TECHNICAL FIELDS SEARCHED (Int. Cl. ³)

The present search report has been drawn up for all claims

| Place of search | Date of completion of the search | Examiner |
|---|---|---|
| THE HAGUE | 25-06-1984 | RIEB K.D. |